Europäisches Patentamt

European Patent Office

Office européen des brevets

(11) Veröffentlichungsnummer: **0 221 277**
**A2**

(12) # EUROPÄISCHE PATENTANMELDUNG

(21) Anmeldenummer: 86112053.3

(22) Anmeldetag: 01.09.86

(51) Int. Cl.⁴: **C 09 B 61/00**
A 23 L 1/275, A 23 J 3/00
A 61 K 9/00

(30) Priorität: 10.09.85 DE 3532129

(43) Veröffentlichungstag der Anmeldung:
13.05.87 Patentblatt 87/20

(84) Benannte Vertragsstaaten:
AT BE CH DE FR GB IT LI NL SE

(71) Anmelder: BAYER AG
Konzernverwaltung RP Patentabteilung
D-5090 Leverkusen 1 Bayerwerk(DE)

(72) Erfinder: Hegasy, Ahmed, Dr.
Tempelhofer Strasse 57
D-5090 Leverkusen 1(DE)

(72) Erfinder: Winter, Manfred, Dr.
Roggendorfstrasse 49
D-5000 Köln 80(DE)

(54) Beta-Carotin enthaltende Gelatine.

(57) Die Erfindung betrifft eine Kombination von β-Carotin und Eisenoxiden als lichtechtes, opakes Färbemittel von Gelatine und ihre Verwendung in Lebensmitteln und Arzneimitteln, insbesondere in der Hülle von Weichgelatinekapseln.

EP 0 221 277 A2

BAYER AKTIENGESELLSCHAFT

Konzernverwaltung RP
Patentabteilung

5090 Leverkusen,Bayerwer

KS/Brü

## β-Carotin enthaltende Gelatine

Die Erfindung betrifft eine Kombination von β-Carotin und Eisenoxiden als lichtechtes, opakes Färbemittel von Gelatine und ihre Verwendung in Lebensmitteln und Arzneimitteln, insbesondere in der Hülle von Weichgelatinekapseln.

Der Lebensmittelfarbstoff β-Carotin (Colour-Index Nr. 40800) ist in Ölen oder Fetten in gelblicher bis orangeroter Farbe löslich. Er ist der Hauptbestandteil des natürlichen Carotins (CI-Nr. 75130) und wird bevorzugt zum Färben von Lebensmitteln wie Margarine oder Fruchtgetränken eingesetzt. Ein wesentlicher Nachteil dieses Farbstoffes ist seine mangelnde Lichtechtheit (vgl. DFG Farbstoff-Kommission, Ringbuch Farbstoffe für Lebensmittel). Im Hinblick auf die Lichtinstabilität wird dort empfohlen, den Farbstoff unter Inertgas kühl und im Dunkeln zu lagern.

Bei der Verwendung von β-Carotin beim Einfärben von Gelatine, insbesondere beim Einfärben von wasserhaltiger Weichgelatine tritt das Problem der mangelnden Lichtechtheit in verstärktem Maße auf. Bereits nach kurzer Zeit beobachtet man ein Verblassen des ursprünglichen Farbtons.

Le A 24 080 Ausland

Diese Instabilität wird noch verstärkt, wenn die Weichgelatine zusätzlich Titandioxid als Opakisierungsmittel enthält. Neben der deutlichen Farbabschwächung tritt bei Titandioxidanwesenheit gleichzeitig ein Farbwechsel von orange nach rosa auf.

Aufgrund dieser Eigenschaften konnte der biologisch gut verträgliche Farbstoff ß-Carotin bisher nicht in befriedigender Weise in Gelatinekapseln eingearbeitet werden. Insbesondere in Fällen, in denen die Färbung der Kapselhülle zusätzlich einen Lichtschutz für empfindliche Wirkstoffe in der Kapselfüllung bewirken sollte, konnte dieser Farbstoff mangels eigener Lichtechtheit nicht eingesetzt werden.

Es wurde gefunden, daß bei der Verwendung einer Kombination von ß-Carotin und Eisenoxiden in Gelatinekapseln opake Färbungen mit hoher Lichtechtheit erhalten werden können. Von besonderem Interesse ist eine Kombination von 0,02 bis 0,5 Gew.%, insbesondere 0,05 bis 0,3 Gew.% ß-Carotin (bezogen auf das Gewicht der Gelatinemasse) und 0,06 bis 0,5 Gew.%, insbesondere 0,15 bis 0,4 Gew.% Eisenoxid (bezogen auf das Gewicht der Gelatinemasse). Das erfindungsgemäße kombinierte Färbemittel enthält somit 1 - 25 Gew.Teile ß-Carotin und 3 - 25 Gew.Teile Eisenoxid.

Es ist bereits bekannt, Eisenoxide und/oder bestimmte Lebensmittelfarbstoffe wie Gelb-orange S (CI Nr. 15985) als Färbe- und Lichtschutzmittel in Weichgelatine einzuarbeiten (vgl. DE-OS 22 09 526). Zur Erreichung eines optimalen Lichtschutzes sind häufig jedoch größere Mengen von etwa 1 bis 2 Gew.% Eisenoxiden notwendig. In einigen Ländern ist die Menge an Eisen in Form von Eisenoxiden, die pro Tag einem Patienten appliziert werden darf, durch

Le A 24 080

behördliche Auflagen limitiert. Zum Beispiel beträgt die zulässige Obergrenze für die Applikation von Eisen in Form von Eisenoxiden in USA 5 mg pro Tag und Patient. Auch die behördliche Zulassung von Lebensmittelfarbstoffen, insbesondere die Zulassung von Vertretern von Azofarbstoffen ist in einigen Ländern mit beschränkenden Auflagen versehen, da einzelne Vertreter dieser Farbstoffe im Verdacht stehen, Nebenwirkungen, wie z.B. Allergien, auszulösen.

Vorteile der erfindungsgemäßen Kombination liegen in der sehr guten Verträglichkeit des ß-Carotins, für welches praktisch keine einschränkenden Auflagen in den einzelnen Ländern bestehen und in der durch die Erfindung ermöglichten signifikanten Reduzierung des Gehaltes an Eisenoxiden, wobei gleichzeitig eine hohe Lichtechtheit und eine optimale Lichtschutzwirkung gewährleistet ist. Gelatinekapseln, die die erfindungsgemäße Kombination enthalten, können mehrfach täglich eingenommen werden, ohne daß der Patient nicht mehr tollerable Eisenmengen aufnimmt. Gleichzeitig können sie über lange Zeiträume gelagert werden ohne ihre ursprüngliche Farbe zu verändern oder in ihrer Lichtschutzwirkung nachzulassen.

Durch entsprechende Variation der Menge des erfindungsgemäß eingesetzten ß-Carotins und der Menge und Art der eingesetzten Eisenoxide lassen sich opake, lichtstabile Gelatinekapseln mit hervorragender Lichtschutzwirkung für die Kapselfüllung in verschiedenen Farbtönen herstellen, z B. gelbe, orangene, rote oder braune Kapseln.

Zum Nachweis der Lichtechtheit werden die erfindungsgemäßen Kapseln gemäß Beispiel 2  24 Std. mit Xenonlicht bestrahlt. Sie zeigen nach diesem Zeitraum keine signifikante Farbveränderung oder ein Verblassen des ursprünglichen Farbtons.

Le A 24 080

Zum Nachweis der lichtschützenden Wirkung wurden die erfindungsgemäßen Kapseln mit einer Lösung des sehr lichtempfindlichen Dihydropyridin-Derivates Nifedipin, gemäß Beispiel 2, gefüllt und ebenfalls im Xenontest belichtet. Nach 24 Std. Belichtungszeit war keine signifikante Zersetzung des Nifedipins festzustellen. Auch andere Dihydropyridine können so vor der Zersetzung durch Lichteinwirkung geschützt werden, z. B. Nisoldipin.

Im folgenden wird beispielhaft die Herstellung einiger Gelatinekapseln beschrieben, die die erfindungsgemäße Farbstoffkombination enthalten:

Beispiel 1    (Vergleichsbeispiel ohne Eisenoxid)

Einfärbung mit feingemahlenem ß-Carotin.

0,100 kg ß-Carotin reinst werden in einer Mischung aus 0,275 kg Glycerin und 0,100 kg Wasser und 0,025 kg Gelatine suspendiert. Die Suspension wird zu 500 ml Glasperlen in einer Perlmühle zugegeben und das ß-Carotin feinst zermahlen. Es entsteht eine rote gleichmäßige Suspension.

Die Suspension wird von den Glasperlen abgetrennt. Diese Farbmischung gibt man zu 42 kg Gelatine, 28 kg Glycerin und 30 kg Wasser. Daraus werden Weichgelatinekapseln mit rötlich oranger Farbe hergestellt im Format 6 minims oblong (1 minim entspricht 0,06 ml) mit einem Hüllengewicht von ca. 210 mg. Als Kapselfüllung werden 377 mg einer Lösung von 3 Gew. Teilen Nifedipin, 94 Gew. Teilen Polyethylenglykol 400, 8 Gew. Teilen Wasser und 8 Gew. Teilen Glycerin pro Kapsel abgefüllt.

Le A 24 080

Beispiel 2

Einfärbung mit fein gemahlenem ß-Carotin und Eisenoxid.

Analog Beispiel 1 wird eine feingemahlene Suspension von ß-Carotin hergestellt. Zur Suspension gibt man 0,5 kg gelbes Eisenoxid, 42 kg Gelatine, 28 kg Glycerin und 30 kg Wasser. Die Gelatinemasse ist opak und hat eine orange Farbe. Die Masse wird zu Kapseln verarbeitet, mit dem selben Inhalt wie Beispiel 1.

Beispiel 3

Einfärbung mit wasserlöslichen ß-Carotin-Zubereitungen und Eisenoxid.

In einem Behälter werden 28 kg Glycerin, 5 kg Wasser und 5 kg Gelatine auf 60°C erwärmt. 1 kg von handelsüblichem sogenannten wasserlöslichen ß-Carotin (mit 10 % ß-Carotin und 90 % Zuschlagstoffen) sowie 0,1 kg Eisenoxid gelb werden eingearbeitet. Dann gibt man weitere 25 kg Wasser und 37 kg Gelatine hinzu. Daraus werden Kapseln hergestellt analog Beispiel 1.

Beispiel 4

Analog Beispiel 3 werden Kapseln mit 5 mg Nisoldipin pro Kapsel hergestellt, indem 226 mg einer Mischung aus 2,5 Gew.Teilen Nisoldipin mit 95 Gew.Teilen Polyethylenglykol 400 und 6 Gew.Teilen Wasser und 10 Gew.Teilen Glycerin verkapselt wird. Das Kapselformat ist 4 minims.

Beispiel 5

Einfärbung mit einer Lösung von ß-Carotin in organischen Lösungsmitteln sowie mit Eisenoxid.

0,1 kg ß-Carotin wird in 5 kg Trichlorethylen gelöst. Die Lösung wird zu einer auf 60°C erwärmten Mischung aus

Le A 24 080

0,2 kg gelbem Eisenoxid, 28 kg Glycerin, 5 kg Wasser und 5 kg Gelatine gegeben.

Diese ß-Carotin-Lösung wird homogenisiert, z.B. durch ein hochtouriges Rührwerk. Es entsteht eine gleichmäßige homogene farbige Suspension. Diese wird auf ca. 60°C gehalten und das Trichlorethylen entfernt. Dann gibt man 37 kg Gelatine und 25 kg Wasser zu und verarbeitet die Masse zu Weichgelatinehüllen analog Beispiel 1.

Beispiel 6

Kapseln mit 5 mg Nisoldipin

In einem Kessel werden unter Lichtschutz 101,6 kg Polyethylenglykol 400 auf ca. 60°C erwärmt. Darin löst man 1,334 kg Nisoldipin. Dann werden 5 kg Wasser, 5 kg Glycerin und 0,066 kg Pfefferminzöl zugegeben. Die Lösung wird abgekühlt und filtriert. Diese Wirkstofflösung wird in Weichgelatinekapseln mit je 424 mg Lösung entsprechend 5 mg Wirkstoff verkapselt. Die Kapselhülle wird hergestellt aus 42,85 kg Gelatine, 26,70 kg Glycerin (Eur. Pharmacopoe), 0,93 kg Titandioxid, 0,93 kg einer 10 %igen ß-Carotin-Dispersion, 0,07 kg Eisenoxyd rot und 28,50 kg Wasser hergestellt.

Beispiel 7

Kapseln mit 2,5 mg Nisoldipin

Eine Wirkstofflösung analog Beispiel 6 wird hergestellt. Die Lösung wird verkapselt zu je 212 mg Lösung pro Kapsel (Format 3 minims).Die Kapselhülle besteht aus 42,6 kg Gelatine, 26,6 kg Glycerin, 0,93 kg Titandioxid, 1,86 kg einer 10 %igen ß-Carotin-Dispersion, 0,14 kg Eisenoxid gelb und 27,0 kg Wasser.

Le A 24 080

Beispiel 8

Kapseln mit 10 mg Nifedipin

Eine Masse für die Kapselhülle wird hergestellt aus 40,1 kg Gelatine; 28,9 kg Glycerin; 28,9 kg Wasser; 1,6 kg einer 10 %igen ß-Carotin-Dispersion; 1,07 kg Titandioxid und 0,06 kg Eisenoxid gelb. Eine Wirkstofflösung bestehend aus 2,94 kg Nifedipin; 94,21 kg Polyethylenglykol 400; 6 kg wasserfreies Glycerin; 10 kg Wasser; 0,15 kg Saccharin Natrium und 0,2 kg Pfefferminzöl wird unter Lichtschutz hergestellt. Die Wirkstofflösung wird zu 386 mg einverkapselt in der angegebenen Masse für Kapselhülle.

Beispiel 9

Eine zweifarbige Kapsel mit 20 mg Nifedipin Zwei verschiedene Gelatinemassen werden hergestellt. Masse A ist mit der Masse in Beispiel 8 identisch. Masse B besteht aus 42,2 kg Gelatine, 28,7 kg Glycerin; 28,7 kg Wasser; 0,60 kg rotem Eisenoxid und 0,17 kg Titandioxid.

Eine Wirkstofflösung wird hergestellt aus 4,6 kg Nifedipin; 96,84 kg Polyethylenglykol 400, 6,2 kg Glycerin wasserfrei; 6,0 kg Wasser und 1,6 kg Pfefferminzöl. Die Lösung wird zu 495 mg in Kapseln der Größe 8 minims verkapselt. Dabei wird eine Hüllenhälfte aus Masse A, die andere Hälfte aus Masse B benutzt.

Le A 24 080

Patentansprüche:

1. Opakes, lichtechtes Färbemittel für Gelatine, enthaltend eine Kombination von 1 bis 25 Gew.-Teilen β-Carotin und 1 bis 25 Gew.-Teilen Eisenoxide.

2. Kombiniertes Färbemittel gemäß Anspruch 1, dadurch gekennzeichnet, daß es 3 bis 18 Gew.-Teile β-Carotin und 1 bis 6 Gew.-Teile Eisenoxid enthält.

3. Verwendung von Färbemitteln gemäß Anspruch 1 bei der Herstellung von lichtechten und lichtschützenden Gelatinekapseln.

4. Verwendung von Färbemitteln gemäß Anspruch 1 als Lichtschutzmittel in Gelatinehüllen.

5. Herstellung von lichtecht gefärbten und lichtschützenden Gelatinekapseln, dadurch gekennzeichnet, daß man 0,02 bis 0,5 Gew.% β-Carotin (bezogen auf das Gewicht der Gelatinemasse) und 0,06 bis 0,5 Gew.% Eisenoxid (bezogen auf das Gewicht der Gelatinemasse) in ein Gemisch von Gelatine, Glycerin und Wasser homogen einarbeitet und diese gefärbte Gelatinemasse nach üblichen Methoden zu Kapselhüllen verarbeitet.

6. Verfahren gemäß Anspruch 5 zur Herstellung von Weichgelatinekapseln.

Le A 24 080